# EUROPÄISCHE PATENTANMELDUNG

(11) **EP 2 289 435 A1**
(43) Veröffentlichungstag der Anmeldung: **02.03.2011**
(21) Anmeldenummer: 09010988.5
(22) Anmeldetag: 27.08.2009
(51) Int. Cl.: A61B 17/225, A61H 23/00, B06B 1/06, H01L 41/083, A61B 17/22, A61B 17/92, A61N 7/02

(54) **Druckwellengerät zur Behandlung des menschlichen oder tierischen Körpers mit Piezolagenstapel**

(71) Anmelder: STORZ MEDICAL AG, 8274 Tägerwilen (CH)
(72) Erfinder: Marlinghaus, Ernst H., Dr., 8598 Bottighofen/TG (CH); Katona, Josef, 78315 Radolfzell (DE); Heine, Gerold, Dr., 78337 Öhningen (DE)
(74) Vertreter: Szynka, Dirk

(57) **Zusammenfassung**

Die Erfindung betrifft ein Gerät (1) zur Behandlung des menschlichen oder tierischen Körpers mit mechanischen Druckwellen unter Verwendung eines Piezolagenstapels (5) mit Lagenschichtdicken von höchstens 150 µm.

## Beschreibung

Die vorliegende Erfindung betrifft das Gebiet der Druckwellenbehandlung des menschlichen oder tierischen Körpers.

Druckwellen, d. h. mechanische und gelegentlich auch als "akustisch" bezeichnete Wellen werden in verschiedener Weise zur therapeutischen Behandlung eingesetzt. Besonders wichtig und historisch betrachtet auch Ausgangspunkt der Entwicklung ist die Stoßwellenlithotripsie, also die Zerkleinerung von Körperkonkrementen, insbesondere Steinen, mit fokussierten Druckwellen großer Amplituden und mit steilen Anstiegsflanken. Hier werden einzelne Pulse auf das Konkrement gerichtet, deren erste, einer Kompression entsprechende "Halbwelle" hinsichtlich Flankensteilheit und Amplitude sowie therapeutischer Wirkung dominiert, wohingegen bereits die nächste nachfolgende Halbwelle, die einer Expansion entspricht, deutlich weniger ausgeprägt ist. Solche Pulse werden regelmäßig wiederholt angewendet.

Vergleichbare Verfahren mit Stoßwellen oder nicht mehr als eigentliche Stoßwellen zu bezeichnenden Druckwellen sind auch für andere Indikationen bekannt, etwa zur Beaufschlagung von schlecht heilenden Knochenbrüchen. Dabei werden auch nicht fokussierte Stoßwellen und Druckwellen eingesetzt. "Stoßwellen" sollen hier als Sonderfall der "Druckwellen", also von diesem Begriff mit inbegriffen, betrachtet werden.

Daneben betrifft die Erfindung aber auch Druckwellentherapien mit eigentlichen, also fortgesetzt oszillierenden Wellen. Diese können in nicht fokussierter, aber auch in fokussierter Weise, eingesetzt werden, etwa zur Erwärmung von Körpergewebe, so zur sog. thermischen Ablation von Tumoren.

Die wesentlichen Frequenzen liegen bei den genannten Therapien über der Hörschwelle; es handelt sich also um Ultraschallverfahren.

Bekannte Techniken zur Erzeugung von Druckwellen im Bereich der Lithotripsie sind elektrische Entladungen in Wasser und auch Felder von flächigen piezoelektrischen Elementen, die an Wasser ankoppeln. Auch durch das Aufeinanderprallen eines beschleunigten Schlagteils und eines Prallkörpers lassen sich Druckwellen erzeugen und sowohl zur Lithotripsie als auch für andere Indikationen verwenden.

Der vorliegenden Erfindung liegt das technische Problem zugrunde, ein vorteilhaftes Druckwellengerät zur Behandlung des menschlichen oder tierischen Körpers von außen, also durch Auflegen auf die Körperoberfläche, anzugeben.

Die Erfindung richtet sich auf ein Druckwellengerät zur Behandlung des menschlichen oder tierischen Körpers mit einem Piezolagenstapel zur Erzeugung von mechanischen Druckwellen, einer auf den zu behandelnden Körper aufzulegenden Applikatorfläche zur Einkopplung der mechanischen Druckwellen in den Körper und einer mit einem der Applikatorfläche entgegengesetzten Ende des Piezolagenstapels verbundenen seismischen Masse, wobei der Piezolagenstapel einen Schichtaufbau zeigt, dessen piezoelektrische Schichtdicken höchstens 150 µm betragen.

Bevorzugte Ausgestaltungen der erfindungsgemäßen Vorrichtung und ihrer Verwendung sind in den abhängigen Ansprüchen angegeben. Die darin enthaltenen Merkmale und auch die Offenbarung der folgenden Beschreibung sind grundsätzlich im Hinblick auf beide Erfindungskategorien zu verstehen, ohne dass hierzwischen immer im Einzelnen explizit unterschieden wird. Rein vorsorglich wird angemerkt, dass sich die Offenbarung auch auf ein Behandlungsverfahren und eine Verwendung zur Behandlung erstreckt.

Die Erfinder schlagen die Verwendung eines piezoelektrischen Antriebs zur Erzeugung von Druckwellen in einem Druckwellengerät zur extrakorporalen Behandlung vor. Bislang sind solche Geräte mit den bereits erwähnten ballistischen Mechanismen betrieben worden, wobei pneumatische Systeme zur Beschleunigung eines Schlagteils oder Projektils eingesetzt wurden.

Dabei schlagen die Erfinder statt der (bei klassischen Lithotriptern bekannten) flächigen Felder von Piezoelementen einen Stapel aus einer großen Zahl piezoelektrischer Lagen vor. Die jeweils sehr begrenzten Hubwege, d. h. piezoelektrischen Extensionen, addieren sich entsprechend der Lagenzahl in dem Stapel zu einem für die gewünschte Anwendung geeigneten Wert auf. Gleichzeitig lassen sich in dieser Form geeignete Energiewerte erzeugen, ohne das Gerät durch die flächige Verteilung einer größeren Zahl von einzelnen Piezoelementen (statt der Stapelanordnung) besonders großvolumig und damit nur schlecht für eine extrakorporale Anwendung geeignet gestalten zu müssen.

Zudem sollen besonders dünne piezoelektrische Lagen eingesetzt werden, nämlich mit Schichtdicken von höchstens 150 µm, vorzugsweise höchstens 140 µm, 130 µm, 120 µm, 110 µm oder 100 µm. Zwar sind solche Piezolagenstapel aufwändiger herzustellen, jedoch hat es sich als sehr vorteilhaft herausgestellt, mit entsprechend kleineren Spannungen arbeiten zu können. Da nämlich die piezoelektrische Extension mit der erzielten Feldstärke korreliert, können ohne Extensionseinbußen kleine Versorgungsspannungen eingesetzt werden, wenn die Lagendicke entsprechend geringer wird. Kleine Versorgungsspannungen verringern aber nicht nur den technischen Aufwand für eine elektrische Versorgung des erfindungsgemäße Geräts; sie verringern vor allem die zur Einhaltung der bei medizinisch-technischen Geräten wesentlichen Sicherheit notwendigen Vorkehrungen und tragen damit insgesamt zur Kosteneffzienz bei.

Der Piezolagenstapel wird an einem Ende mit einer sog. seismischen Masse verbunden, also einer im Verhältnis zur Masse des Applikators und zusätzlich zur Masse des Piezolagenstapels selbst großen trägen Masse. Diese sorgt für eine "Abstützung" des Piezolagenstapels bei seiner elektrischen Beaufschlagung und Extension infolge der aus der Trägheit der seismischen Masse resultierenden reaktiven Beschleunigungskraft.

An dem entgegengesetzten Ende des Piezolagenstapels wird die erzeugte Druckwelle "abgegriffen", was in unterschiedlicher Form geschehen kann, vgl. die folgenden Erläuterungen. Über eine Applikatorfläche, die zur Behandlung auf den Körper aufgelegt wird, wird die mechanische Druckwelle in den Körper eingekoppelt.

Damit ist das erfindungsgemäße Gerät kostengünstig und je nach Bedarf auch besonders kompakt zu konstruieren und zu produzieren. Es zeigen sich im Vergleich zu den beschriebenen ballistischen Geräten zudem Vorteile hinsichtlich der Geräuschentwicklung und des Verschleißes.

Zwei Möglichkeiten der Erzeugung der letztlich in den zu behandelnden Körper einzukoppelnden Druckwelle kommen bevorzugt in Betracht:
Bei der ersten bevorzugten Variante ist ein eigentlicher Applikator im Sinne eines eigenständigen Bauteils vorgesehen, der die Applikatorfläche aufweist und ein von dem Piezolagenstapel körperlich getrenntes Element bildet. Hierbei ist anzumerken, dass im allgemeinen Sinn der Erfindung die Applikatorfläche auch durchaus eine Fläche des Piezolagenstapels selbst sein könnte. Bei der hier betrachteten bevorzugten Variante mit separatem Applikator(-körper) ist dieser zwar körperlich von dem Piezolagenstapel getrennt, aber mechanisch daran gekoppelt, sodass Kräfte übertragen werden können. Insbesondere kann der Applikator über eine elastische Einrichtung, also eine Feder im allgemeinsten Sinn einschl. von Elastomerelementen, auf den Piezolagenstapel aufgedrückt sein. Auf diese Variante wird noch weiter eingegangen.

Die zweite bevorzugte Variante sieht ebenfalls einen eigenen Applikator vor, der allerdings mechanisch von dem Piezolagenstapel entkoppelt ist. Zusätzlich ist hier ein Schlagteil vorgesehen, das durch die piezoelektrische Extension beschleunigt wird und ähnlich wie bei den ballistischen Mechanismen auf den Applikator als Prallkörper trifft. Hier ersetzt der erfindungsgemäße Piezolagenstapel also den pneumatischen Antrieb zur Beschleunigung eines Projektils, während bei der zuvor angesprochenen Variante kein Schlagteil oder Projektil vorgesehen ist.

Die seismische Masse kann ein Gehäuseteil sein oder auch durch das gesamte Gerätegehäuse gebildet sein.

Der Applikator kann im Rahmen der Erfindung durchaus zusätzliche Aufgaben übernehmen, insbesondere die Druckwellen fokussierende oder defokussierende Eigenschaften haben. So kann es sich bei dem Applikator beispielsweise um einen Rotationsellipsoiden handeln, vgl. DE 10 2007 013 288. Die Applikatorfläche kann zudem (selbst ohne separaten Applikatorkörper) gewölbt sein.

Vorzugsweise ist der Piezolagenstapel eingespannt, auch bei der Variante mit davon getrenntem Applikator. Diese Einspannung muss natürlich elastisch nachgiebig sein, um die gewünschte piezoelektrische Extension zuzulassen. Vorzugsweise ist die Stärke der Vorspannung, d. h. die haltende Kraft bezogen auf die Querschnittsfläche, einstellbar. Typische Werte der flächenbezogenen Vorspannung liegen bei etwa 10 MPa - 25 MPa, vorzugsweise 12 MPa - 22 MPa und besonders bevorzugter Weise bei 14 MPa -19 MPa.

Eine günstige Ausführung des Piezolagenstapels ist zylindrisch, wobei die Zylinderachse senkrecht zu den Piezolagen liegt, diese also jeweils kreisscheibenförmig aufeinander geschichtet sind. Diese Bauform lässt sich gut in handliche Gehäuse einbauen und erlaubt eine große Zahl von Einzellagen.

Das erfindungsgemäße Gerät weist vorzugsweise ein Handteil auf, also eine von der Bedienungsperson mit der Hand oder den Händen zu bewegende und zu handhabende Einheit. Dabei kann das Druckwellengerät vollständig durch das Handteil gebildet sein oder ein zusätzliches Basisteil aufweisen, das mit dem Handteil verbunden ist. Das Basisteil kann insbesondere Versorgungscharakter haben, also insbesondere die elektrische Versorgung für den Piezolagenstapel in dem Handteil gewährleisten. Ferner kann das Basisteil Einstellmöglichkeiten für verschiedene Parameter bieten.

Bevorzugt ist ferner ein Batteriebetrieb, insbesondere bei einem Handteil, das das gesamte erfindungsgemäße Gerät ausmacht. Hier zeigen sich die Vorteile der Erfindung, nämlich eine kompakte und leichte Bauform mit geringem apparativen Aufwand für die Erzeugung der Druckwellen, besonders deutlich.

Vorzugsweise sind die Lagen des Piezolagenstapels so ausgelegt, dass sich die elektrische Versorgung auf Spannungen bis in den Bereich von unter etwa 350 V, vorzugsweise 300 V, besser 250 V, unter etwa 200 V, beschränken kann, um die zugelassenen Maximalauslenkungen zu erzielen. Daraus ergeben sich dann keine über normale Haushaltsspannungen hinausgehenden Anforderungen. Ein weiterer Vorteil des piezoelektrischen Prinzips gemäß der Erfindung liegt in relativ hohen erreichbaren Frequenzen. Üblicherweise erfolgt die Beaufschlagung des Körpers durch die Druckwellen nämlich pulsweise, also nicht mit einer kontinuierlichen Druckwelle. Die Wiederholfrequenzen, die sich mit den bekannten pneumatischen Antrieben erzielen lassen, liegen typischerweise im einstelligen und niedrigen zweistelligen Hertzbereich. Piezoelektrische Antriebe lassen jedoch deutlich höhere Frequenzen zu, insbesondere auch weit über 100 Hz. Damit lässt sich eine bestimmte Zahl von Einzelpulsen mit bestimmten Eigenschaften in kürzerer Zeit einbringen, sodass die Behandlungsdauer insgesamt verringert werden kann.

Die bereits mehrfacht erwähnte seismische Masse ist vorzugsweise austauschbar. Damit können durch verschieden große Massen die Impulsformen beeinflusst und unterschiedliche Anforderungen für verschiedenen Anwendungsfälle berücksichtigt werden.

Eine als Ausführungsbeispiel näher dargestellte günstige Bauform sieht eine Verbindung zwischen der seismischen Masse und einer Hülse vor, wodurch ein innerer Hohlraum entsteht. Die seismische Masse kann dabei als Gehäuseteil ausgeführt sein, wie das Ausführungsbeispiel zeigt. In dem Hohlraum kann der Piezolagenstapel angeordnet sein und bei der erwähnten ersten Variante elastisch über den Applikator gegen die seismische Masse gedrückt werden. Dazu stützt sich der Applikator günstigerweise elastisch an einem Bund der Hülse ab. Der Applikator durchsetzt ferner eine Öffnung in der Hülse, die mit dem Hohlraum kommuniziert und an deren der seismischen Masse entgegengesetztem Ende vorgesehen ist. Die erwähnte elastische Beaufschlagung kann insbesondere durch einen Elastomeing zwischen dem Applikator und dem erwähnten Bund der Hülse erfolgen.

Bei der erwähnten zweiten Variante kann die Hülse ebenfalls eine Öffnung aufweisen, in der der Applikator angeordnet ist, wobei er sich elastisch gegen einen Bund um die Öffnung herum abstützt. Hier ist der Applikator allerdings entkoppelt von einem Schlagteil angeordnet, das beispielsweise eine weitere Öffnung in einem Teil der Hülse und innerhalb des Hohlraums durchsetzt und (quasi anstelle des Applikators bei der ersten Variante) elastisch an einem dortigen Bund abgestützt und auf den Piezolagenstapel aufgedrückt sein kann.

Die Ausführung der bereits zuvor erwähnten elastischen Beaufschlagung des Piezolagenstapels in Form eines Elastomerrings, insbesondere O-Rings, ist auch unabhängig von diesen Bauformen bevorzugt.

Schließlich ist die Verwendung des Geräts gerade bei der Behandlung von Körperweichgewebe, beispielsweise Muskeln oder Sehnen, bevorzugt. Dies schließt die Behandlung knochennaher Bereiche und eine Stoßwellenakupunktur ein. Typische Indikationen sind Ansatztendinosen und andere Anwendungen in Orthopädie und Chirurgie wie Kalkschultern, Fersenschmerzen, Pseudarthrosen, aber auch Muskelzerrungen. Weitere Indikationen gibt es in der Neurologie, etwa die Verbesserung der Motorik nach Schlaganfällen, die Behandlung von Spasmen nach Traumen sowie Poly-Neuropathien. In der Urologie können etwa chronische Beckenbodenschmerzen behandelt werden; in der Angiologie / Dermatologie und Chirurgie außerdem Narben oder Hautverbrennungen behandelt sowie eine Verbesserung der Wundheilung erzielt werden.

Im Folgenden wird die Erfindung anhand von Ausführungsbeispielen näher erläutert, wobei die einzelnen Merkmale auch in anderen Kombinationen erfindungswesentlich sein können und sich implizit auf alle Kategorien der Erfindung beziehen.
- Figur 1: zeigt eine perspektivische Darstellung eines Handteils eines erfindungs- gemäßen Geräts als erstes Ausführungsbeispiel.
- Figur 2: zeigt einen Seitenaufriss dazu.
- Figur 3: zeigt den Seitenaufriss aus Figur 2 mit eingezeichneter Schnittlinie B-B zu Figur 4 und
- Figur 4: zeigt den in Figur 3 eingezeichneten Schnitt B-B.
- Figur 5: zeigt eine perspektivische Darstellung eines Handteils eines erfindungs- gemäßen Geräts als zweites Ausführungsbeispiel.
- Figur 6: zeigt einen Seitenaufriss dazu mit eingezeichneter Schnittlinie A-A zu Fi- gur 7.
- Figur 7: zeigt den in Figur 6 eingezeichneten Schnitt A-A.

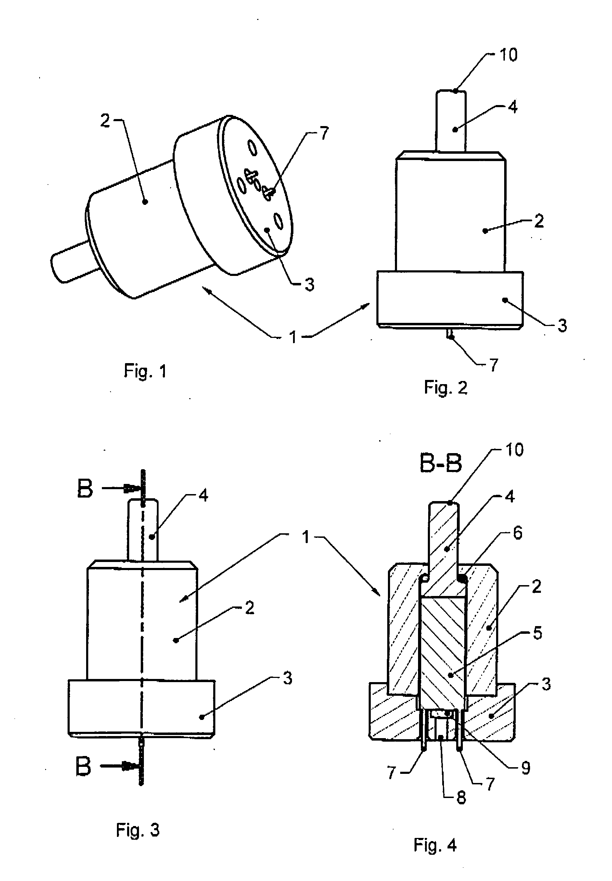

In den Figuren 1 - 4 sieht man ein Handteil eines erfindungsgemäßen Behandlungsgeräts. Zusätzlich gehört zu dem erfindungsgemäßen Gerät dieses Ausführungsbeispiels eine elektrische Versorgung, die über nicht dargestellte Kabel an das dargestellte Handteil 1 angeschlossen werden kann.

Das Handteil 1 weist ein Gehäuse aus einer Hülse 2 und einem seismischen Massekörper 3 auf. Der Massekörper 3 bildet einen Gehäuseboden, in den die Hülse 2 eingesetzt ist, wie insbesondere Figur 4 im Schnitt zeigt. Der Massekörper 3 ist dabei durch Schrauben befestigt, die in Figur 1 auf der Stirnfläche des Massekörpers 3 eingezeichnet sind. Sie durchsetzen den Massekörper 3 und verankern diesen in der Hülse 2. An einem dem Massekörper 3 entgegengesetzten Ende, nämlich in den Figuren 2 - 4 oben, weist die Hülse 2 eine zentrische Öffnung auf, die von einem Applikator 4 in Form eines Stößels durchsetzt ist. Die Hülse 2 und der Massekörper 3 bilden dabei einen im Wesentlichen zylindrischen Innenraum, in den ein Piezolagenstapel 5 eingesetzt ist, den Figur 4 zeigt. Der Innenraum ist nach oben durch den Applikator 4 verschlossen, der sich dabei über einen Elastomer-O-Ring 6 an einem Bund der Hülse 2 abstützt und zu diesem Zweck eine stempelartige Verdickung aufweist. Im unteren Bereich ist der Innenraum durch den Massekörper 3 abgeschlossen, wobei zwei in den Figuren 1 und 4 erkennbare elektrische Durchführungen 7 vorgesehen sind, über die der Piezolagenstapel 5 elektrisch angeschlossen werden kann. Zwischen diesen elektrischen Durchführungen 7 befindet sich ein Innengewindeloch 8, das durch eine eingelegte Scheibe 9 von dem Piezolagenstapel 5 getrennt ist. In das Innengewindeloch 8 kann eine Einstellschraube eingeschraubt werden, um über die Scheibe 9 gegen die Elastizität des O-Rings 6 den Piezolagenstapel 5 unter eine einstellbare Vorspannung von etwa 15 MPa - 18 MPa zu setzen. Abgesehen von den elektrischen Durchführungen 7 ist der Aufbau im Übrigen weitgehend zylindersymmetrisch. Die Darstellung ist ungefähr maßstäblich, und zwar in realer Größe. Das Handteil ist damit also vergleichsweise klein. Nach Anschluss einer elektrischen Versorgung, die einstellbare Spannungspulse an die elektrischen Durchführungen 7 gibt, können mit dem Handteil 1 des Geräts mechanische Druckwellen erzeugt werden. Dabei expandiert der Piezolagenstapel 5 infolge der Spannungspulse, wobei die Spannungen jeweils mit alternierender Polarität an seine Lagen angelegt sind und die piezoelektrische Polarisation der Lagen ebenfalls alternierend ist. Damit können benachbarten Lagen jeweils eine Elektrode, also leitende Zwischenschicht, teilen. Insoweit erzeugt ein Spannungspuls von maximal 160 V bei vorliegend 340 Einzellagen aus dem Piezomaterial mit einer ungefähren jeweiligen Lagendicke des Piezomaterials (ohne Leiterschichten) von 80 µm eine piezoelektrische Extension von etwa 40 µm - 45 µm. Diese überträgt sich auf den Applikator 4 und über diesen auf den Körper. Dazu wird die in den Figuren 2 - 4 nach oben weisende Fläche 10 des Applikators 4 als Applikatorfläche 10 auf den Körper aufgelegt.

Die Versorgungspulse können durch transistorschaltergesteuerte Entladung eines zwischen den Pulsen jeweils wieder aufgeladenen Kondensators erzeugt werden, wobei die elektrische Pulsform zusätzlich durch eine Drossel angepasst werden kann.

Die mechanische Pulsform hängt dann zusätzlich von den elastischen Eigenschaften des O-Rings 6 und den Massen des Applikators 4 und des seismischen Massekörpers 3 ab. Letzterer ist durch einfaches Abschrauben austauschbar und erlaubt damit ebenfalls eine Anpassung. Auch der Applikator 4 kann nach Abschrauben des Massekörpers 3 ausgetauscht werden, und zwar nicht nur wegen einer Massenanpassung, sondern auch zur Formanpassung, etwa mit einer konvexen oder konkaven Applikatorfläche oder als Rotationsellipsoid.

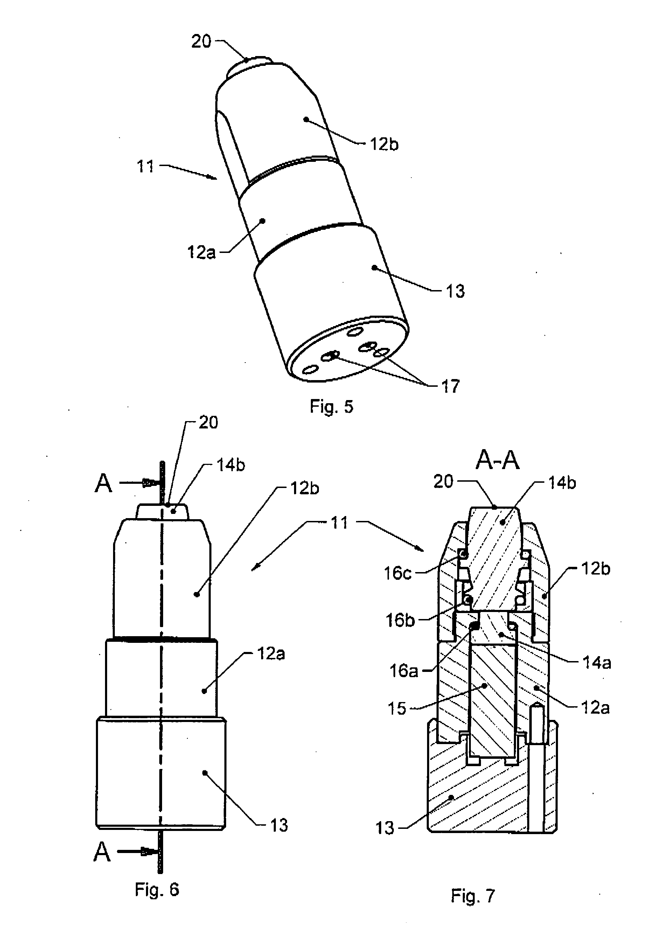

Ein zweites Ausführungsbeispiel findet sich in den Figuren 5 - 7. Die Bezugszeichen sind gegenüber dem ersten Ausführungsbeispiel um 10 erhöht. Die Konstruktion des zweiten Ausführungsbeispiels hat deutliche Ähnlichkeiten mit der des ersten; auf die Besonderheiten wird im Folgenden eingegangen.

Hier ist eine zweiteilige Hülse 12 mit einem in den Figuren unteren Teil 12a und einem oberen Teil 12b vorgesehen. Die beiden Hülsenteile sind miteinander verschraubt, wie Figur 7 zeigt. Dazu ist an einem in den Figuren oberen radial zurückspringenden Ansatz des unteren Hülsenteils 12a ein Außengewinde und im unteren Bereich des oberen Hülsenteils 12b ein Innengewinde vorgesehen.

In Figur 7 ist unten rechts ein Gewindeloch für eine Befestigungsschraube zu sehen, die den drei Befestigungsschrauben des ersten Ausführungsbeispiels, die in Figur 4 nicht im Schnitt dargestellt sind, entspricht.

Ein Piezolagenstapel 15 ist in einem durch die Hülse 12 gebildeten Innenraum, insbesondere innerhalb des unteren Hülsenteils 12a, angeordnet. Diese Anordnung entspricht weitgehend dem ersten Ausführungsbeispiel, wobei hier die Einrichtung zur Spannungseinstellung 8, 9 aus Figur 4 weggelassen ist. Anstelle des Applikators 4 aus dem ersten Ausführungsbeispiel ist hier ein diesem in der Form entsprechendes, aber gewissermaßen mit der oberen Kante des unteren Hülsenteils 12a abgeschnittenes Schlagteil 14a vorgesehen, das sich in gleicher Weise über einen Elastomerring 16a gegen einen Bund um eine Öffnung in dem unteren Hülsenteil 12a abstützt.

Ein weiterer Teil des Hohlraums innerhalb des oberen Hülsenteils 12b ist ausgefüllt von dem eigentlichen Applikator 14b, der eine für sich betrachtet bereits in der Patentanmeldung DE 10 2007 013 288 beschriebene Form eines angenährten Rotationsellipsoiden aufweist. Diese Form dient zur Fokussierung von auf der in den Figuren unteren Stirnfläche erzeugten Druckwellen auf einen Fokuspunkt an oder jenseits der in den Figuren oberen Stirnfläche, nämlich der Applikatorfläche 20. Der obere Fokuspunkt ist der erste der beiden Brennpunkte des Ellipsoiden, der untere Brennpunkt liegt in der unteren Stirnfläche. Die Druckwellenerzeugung erfolgt durch einen Aufprall des Schlagteils 14a auf dieser unteren Stirnfläche, von der das Schlagteil 14a in dem in Figur 7 gezeigten Zustand durch einen sehr kleinen Spalt, der in der Figur nicht erkennbar ist, getrennt ist.

Der Applikator 14b ist über Elastomerringe 16b und 16c gegen einen Bund in dem oberen Hülsenteil 12b nach unten, also zur Seite des Massekörpers 13 bzw. nach oben gegen einen Bund um die Öffnung zur Applikatorfläche 20 hin abgestützt. Der erstgenannte Bund ist durch einen L-Profilring gebildet, der nach Einsetzen des Applikators 14b in den oberen Hülsenteil 12b im Zuge des Zusammenbaus aufgesetzt wird, in diesem Fall zugunsten einer Austauschbarkeit aufgeschraubt wird. Der Applikator 14b kann sich also axial in beide Richtungen etwas bewegen und damit aus seiner Ruhelage durch das Aufschlagen des Schlagteils 14a ausgelenkt werden und abgefangen durch den Elastomerring 16b in diese Ruhelage wieder zurückkehren.

Die Druckwellenerzeugung erfolgt hier also durch Beschleunigung des Schlagteils 14a in Folge einer piezoelektrischen Extension des Piezolagenstapels 15, wobei das Schlagteil 14a durch den Elastomerring 16a deutlich weicher gelagert ist als beim ersten Ausführungsbeispiel. Es kommt also zu einer etwas größeren Bewegung, die abhängig von der eingesetzten Energie in der Größenordnung von 0,05 mm - 0,5 mm liegen kann, und einem Aufprall auf dem Applikator 14b nach Überbrückung des nicht gezeichneten Spalts dazwischen. Der Aufprall erzeugt eine Druckwelle in dem Applikator 14b, die von diesem fokussiert über die Applikatorfläche 20 in den behandelten Körper eingekoppelt wird.

Beide Ausführungsbeispiele zeigen Handteile 1 bzw. 11, die über die Kabelanschlüsse 7 bzw. 17 zum Anschluss an eine Basisstation vorgesehen sind. Die Basisstation kann batteriebetrieben aber auch netzbetrieben sein. Ein Batteriebetrieb ermöglicht auch die Integration der Ansteuerelektronik in das Handteil, sodass das gesamte erfindungsgemäße Druckwellengerät als Handgerät ausgeführt ist. Dies ist nicht dargestellt.

## Patentansprüche

1. Druckwellengerät zur Behandlung des menschlichen oder tierischen Körpers mit
einem Piezolagenstapel (5, 15) zur Erzeugung von mechanischen Druckwellen,
einer auf den zu behandelnden Körper aufzulegenden Applikatorfläche (10, 20) zur Einkopplung der mechanischen Druckwellen in den Körper und
einer mit einem der Applikatorfläche (10, 20) entgegengesetzten Ende des Piezolagenstapels verbundenen seismischen Masse (3, 13),
wobei der Piezolagenstapel (5, 15) einen Schichtaufbau zeigt, dessen piezoelektrische Schichtdicken höchstens 150 µm betragen.

2. Druckwellengerät nach Anspruch 1 mit einem Applikator (4), der die Applikatorfläche (10) aufweist und von dem Piezolagenstapel (5, 15) körperlich getrennt, aber mit diesem mechanisch gekoppelt ist, insbesondere beaufschlagt durch eine elastische Einrichtung (6).

3. Druckwellengerät nach Anspruch 1 mit einem Applikator (14b), der die Applikatorfläche (20) aufweist, von dem Piezolagenstapel (15) körperlich getrennt und von diesem mechanisch entkoppelt ist, und mit einem Schlagteil (14a), das durch eine piezoelektrische Expansion des Piezolagenstapels (15) beschleunigt werden und auf den Applikator (14b) auftreffen kann.

4. Druckwellengerät nach einem der vorstehenden Ansprüche mit einer Vorrichtung (8, 9) zum Einstellen einer Vorspannung des Piezolagenstapels (5).

5. Druckwellengerät nach einem der vorstehenden Ansprüche, bei dem der Piezolagenstapel (5, 15) zylindrisch ist und die Zylinderachse senkrecht zur Ebene der Piezolagen liegt.

6. Druckwellengerät nach einem der vorstehenden Ansprüche mit einem dem Piezolagenstapel (5, 15) und die Applikatorfläche (10, 20) aufweisenden Handteil (1, 11) zur Bewegung und Handhabung durch die Hand einer bedienenden Person.

7. Druckwellengerät nach einem der vorstehenden Ansprüche mit einer Batterieversorgung für die elektrische Ansteuerung des Piezolagenstapels (5, 15).

8. Druckwellengerät nach einem der vorstehenden Ansprüche, bei der der Piezolagenstapel (5, 15) mit Spannungen im Bereich bis höchstens 350 V zur zulässigen Materialauslenkung zu versorgen ist.

9. Druckwellengerät nach einem der vorstehenden Ansprüche, das für Pulswiederholfrequenzen jeweiliger piezoelektrischer Extensionen von über 100 Hz ausgelegt ist.

10. Druckwellengerät nach einem der vorstehenden Ansprüche, bei dem die seismische Masse (3, 13) zur Einstellung der Pulsform der mechanischen Druckwellen austauschbar ist.

11. Druckwellengerät nach einem der vorstehenden Ansprüche, bei dem der Piezolagenstapel (5) durch einen Elastomer-O-Ring (6) elastisch beaufschlagt ist.

12. Druckwellengerät nach einem der vorstehenden Ansprüche, zumindest Anspruch 2 oder 3, mit einer Hülse (2, 12a, b), die mit der seismischen Masse (3, 13) verbunden ist, mit dieser gemeinsam einen Hohlraum definiert und eine an einem der seismischen Masse (3, 13) entgegengesetzten Hülsenende vorgesehene Öffnung aufweist, die mit dem Hohlraum kommuniziert, wobei der Applikator (4, 14b) in der Öffnung und der Piezolagenstapel (5, 15) in dem Hohlraum angeordnet ist.

13. Druckwellengerät nach Anspruch 12, bei dem der Applikator (4) gegen einen Bund der Hülse (2) elastisch abgestützt ist und durch die elastische Abstützung gegen den Piezolagenstapel (5) beaufschlagt ist.

14. Druckwellengerät nach Anspruch 12, bei dem der Applikator (14b) gegen einen Bund eines applikatorseitigen Teils (12b) der Hülse (12) elastisch abgestützt ist und ein Schlagteil (14a) gegen einen Bund eines der seismischen Masse (13) zugewandten Teils (12a) der Hülse (12) elastisch abgestützt und durch die elastische Abstützung (16a) gegen den Piezolagenstapel (15) beaufschlagt ist.
